## EUROPEAN PATENT SPECIFICATION

(19) Europäisches Patentamt
European Patent Office
Office européen des brevets

(11) Publication number: **0 127 658**
**B1**

(12)

(45) Date of publication of patent specification: **14.10.87**

(51) Int. Cl.⁴: **C 12 P 21/06**

(21) Application number: **84900036.9**

(22) Date of filing: **09.12.83**

(86) International application number:
**PCT/DK83/00118**

(87) International publication number:
**WO 84/02351 21.06.84 Gazette 84/15**

(54) **A PROCESS FOR PREPARING RIPE PROTEINS FROM FUSION PROTEINS, SYNTHETIZED IN PRO- OR EUKARYOTIC CELLS.**

(30) Priority: **10.12.82 DK 5493/82**

(43) Date of publication of application:
**12.12.84 Bulletin 84/50**

(45) Publication of the grant of the patent:
**14.10.87 Bulletin 87/42**

(84) Designated Contracting States:
**AT BE CH DE FR GB LI LU NL SE**

(56) References cited:
**EP-A-0 020 290**
**EP-A-0 089 626**

**Hirs, C H W (Ed), Methods in Enzymology, Vol 6, 1967, Academic Press Inc., New York, p 426-36**

**Chemical Abstracts, Vol. 98 (1983), abstract No 212 112, Biochim. Biophys. Acta 1983, 743 (3), 437-47 (Eng)**

(73) Proprietor: **Nordisk Gentofte**
**Niels Steensensvej 1**
**DK-2820 Gentofte (DK)**

(72) Inventor: **CHRISTENSEN, Thorkild**
**Bellisvej 55**
**DK-3450 Allerod (DK)**
Inventor: **BALSCHMIDT, Per**
**Tibberupalle 20**
**DK-3060 Espergaerde (DK)**
Inventor: **DAHL, Hans-Henrik, Marstrand**
**c/o Royal Childrens Hospital Flemington Road**
**Parville VI-3052 (AU)**
Inventor: **HEJNAES, Kim Ry**
**Bredebovej 29, V**
**DK-2800 Lyngby (DK)**

(74) Representative: **Keller, Johanna Carola et al**
**Deichmannhaus am Hauptbahnhof**
**D-5000 Köln 1 (DE)**

EP 0 127 658 B1

Courier Press, Leamington Spa, England.

## Description

The invention relates to a process for preparing ripe proteins from fusion protein, synthetized in pro- or eukaryotic cells.

Proteins and peptides are synthetized in all live cells in which they are essential components. Moreover, certain proteins and peptides, such as the peptide hormones insulin and growth hormone, play an important role in intercellular communications. Other proteins and peptides, such as immunoglobulins and blood clotting factors, are of great importance to the defense mechanisms of higher organisms.

Peptides and proteins consist of short and long chains of amino acids, respectively, and a given protein may include one or more chains. The amino acids are selected from the about 20 normally occurring cellular amino acids. The amino acid sequence in a given protein or peptide chain is specified in the genes of the cell. The genes of the vast majority of organisms are composed of long chains of desoxyribonucleic acid (DNA) consisting of nucleotide triplets (codons) and when a protein is to be synthetized, it takes place through a ribonucleic acid (RNA) copy of the coding DNA region. This so-called messenger RNA is then used by the cell as a pattern for the protein as ribonucleotide triplets (and thus indirectly the corresponding desoxyribonucleotide triplets in the DNA chain) specify specific amino acids in accordance with the genetic code.

The basic molecular-biological principles which, based on DNA, lead to the formation of a protein are well-known to those skilled in the art.

When proteins are synthetized in cells, the product first formed is seldom equal to the final, ripe protein. Most often, e.g. a somewhat longer amino acid chain is synthetized which is then cut to the length of the desired pipe protein, probably enzymatically. The cut amino acid sequences are usually disposed in the N-terminal end of the protein, and are called pro-sequences or signal sequences, according to their function and time of cleavage. Usually, only the ripe protein has full biological activity.

Initiation of the biosynthesis of proteins/peptides takes place by a mechanism causing the N-terminal amino acid in the produced protein or peptide to always be menthionine (in eukaryotic cells) or N-formyl methionine (in bacteria). This N-terminal methionine (or formylmethionine) is often included in a pro-sequence or a signal sequence, which is cleaved later, and therefore is not present in the ripe protein.

Discoveries and progress in recent years within molecular biology have made it possible to transfer genes (DNA segments) *in vitro* from one organism to another, unrelated organism. Thus, e.g. human genes can be transferred to bacteria, such as E. coli. If the human DNA segment is modified so as to resemble bacterial DNA, the bacteria may in certain causes be cused to synthetize a protein identical or closely related to the protein which is coded for by the human DNA segment. Normally, however, great problems are associated with the making bacteria synthetize a product by this genetic engineering which is structurally identical to the desired ripe eukaryotic protein. Usually, the resulting bacterial product is a fusion protein between an amino acid sequence of a bacterial protein and the desired eukaryotic protein or it is the eukaryotic protein provided with an N-terminal formyl methionine group. These products are only rarely further modified in bacteria.

To remedy this situation, it is proposed in the DK patent application No. 1616/81 to couple a bacterial signal sequence directly to a eukaryotic protein, and the bacteria itself will hopefully then cleave off the signal sequence. However, this is not a universally useful method because the cleavage also seems to depend upon the amino acid sequence of the eukaryotic protein.

Further, it is proposed in the DK patent application No. 888/81 to incorporate specific proteolytic or chemical cleavage sites at the border between the bacterial and the eukaryotic part of the protein. The usefulness of this method, however, is limited because the desired ripe eukaryotic protein must not contain the same proteolytic or chemical cleavage site.

The fusion protein may be cleaved chemically at specific amino acids (e.g. after methionine using BrCN). However, this method requires that the specific amino acid in question is not also present in the ripe protein, and the method is vitiated by a certain risk due to the toxicity of BrCN.

When synthetized in bacteria, the newly synthetized protein always contains, as mentioned, N-terminal N-formyl methionine and optionally a pro-sequence which is not present in proteins of eukaryotic cells and must therefore always be removed if the bacterially synthetized protein is to have the same primary amino acid sequence as the desired protein from e.g. humans.

Similarly, proteins formed in eukaryotic cells always contain N-terminal methionine.

In addition, a suitable DNA sequence may be incorporated by genetic engineering in front of the DNA segment coding for the desired protein so that the expressed fusion protein is caused to contain an appropriate N-terminal amino acid sequence. This amino acid sequence may e.g. be selected so that the purification of the fusion protein will be easier, that a desired proteolytic cleavage site results or that the fusion protein is transported out of the bacteria. Such an N-terminal amino acid sequence must of course also be removed to provide the desired, ripe protein.

Generally, it may be said that when the bacteria cannot be caused to produce the desired protein directly, then the resulting fusion protein must be treated enzymatically or chemically so that the additional N-terminal amino acids are removed without the reactions influencing the desired protein.

A process for enzymatic cleavage of a fusion protein is known from the EP Publication No. 0020290. In this process the enzyme collagenase is used for cleaving a bond in the signal or pro-sequence of the fusion protein, viz. the bond Xyz-Gly in which Xyz is a peptide chain incorporated in the pro-sequence. The remaining amino acid or acids in the pro-sequence are the removed enzymatically.

However, it has been found that collagenase and other endopeptidases are deficient in specificity, see Bio-chim. Biophys. Acta, 271 (1972) 133—144. Moreover, the collagenases are active only in connection with proteins of a specifc steric structure, see Peptides 1980, Proceedings of the sixteenth European Peptide Symposium, Ed. K. Brunfeldt (Scriptor Copenhagen 1981)

Proteolytic enzymes (proteases) are characterized in that in certain circumstances they can hydrolyze the peptide bonds between the amino acids in proteins (and peptides). The proteolytic enzymes constitute a very large and heterogeneous group of proteins, both as regards mode of operation and the specificity with which the amino acid sequences are cleaved. In brief summary, the proteolytic enzymes are divided into two main groups: exo- and endoproteases. As indicated by the names, exoproteases cleave off amino acids from the end of the protein chain, whereas endoproteases can directly cleave in the interior of a protein. Aminopeptidases are exoproteases cleaving off amino acids from the N-terminal end of the protein chain. Proteases often have a high degree of specificity as they mainly cleave bonds in specific amino acid sequences. The cleavage is not only dependent upon the amin acid sequence, but also the sterical structure of the substrate may have a great influence on the cleavage rate. The ability of proteases to cleave proteins in a specific manner has been used for a number of years for characterization, analysis and modification of proteins.

The present invention is based on the use of an exo-peptidase which cleaves off the amino acid groups in the pro-sequence of the fusion protein one by one until only the group Y-Pro remains in which Y is an arbitrary amino acid.

The exopeptidase used is an aminopeptidase, and as such leucine aminopeptidase is preferred.

The object of the present invention is to provide a process in which a protein, synthetized by genetic engineering in pro- or eukaryotic cells, can be converted *in vitro* by enyzmatic processes to a protein having an amino acid sequence identical to the selected eukaryotic protein, and thus solve the following two problems in the preparation of ripe proteins:

a) removal of the formyl methionine or the methionine group,

b) release of the ripe protein from the pro-sequence.

According to the invention, proteases are used, and in particular aminopeptidases, for cleaving off, if necessary, formyl methionine and optionally other N-terminal amino acids in a synthetized fusion protein. Methods of Enzymology 11 (1968), p.p 426/36 teaches a process being somewhat similar to that of the invention, i.e. the hydrolyses of small peptides or fractions of peptides, using Lap in general. There is mentioned a decapeptide which is cleaved to a hexapeptide on the one side and "a large peptide" derived from hemologlobin on the other side. This last mentioned peptide was first cleaved by papain, however. The use of Lap for cleaving a prosequence from ripe proteins such as human growth hormone or proinsulin is neither known nor obvious for the skilled artisan in knowledge of this prior art. The invention, however, relates to a process for preparing ripe proteins by enzymatic cleavage of a fusion protein with the amino acid sequence:

$$(Y_m \ldots Y_2{-}Y_1){-}(Pro)_p{-}(X_1{-}X_2 \ldots X_n)$$

in which $(Y_m \ldots Y_2{-}Y_1){-}(Pro)_p$ is the pro-sequence, with the proviso that none of the amino acids $(Y_m \ldots Y_2{-}Y_1)$ can be Pro, and the rest is the ripe protein, m is an integer greater than 2 and Y is an arbitrary amino acid, P is 0 if $X_1$ or $X_2$ is Pro, and 1 if $X_1$ or $X_2$ is different from Pro, X is an arbitrary amino acid, and n is an integer equal to or greater than 4, and the process is characterized by carrying out the enzymatic cleavage by stepwise cleavage off of the amino acid groups $Y_m \ldots Y_2$ if p=1 or $X_1$=Pro, or the groups $Y_m \ldots Y_2{-}Y_1$ if $X_2$=Pro, by means of an aminopeptidase, and then the two amino acids $Y_1$-Pro if p=1 are cleaved off enzymatically in one or two steps in a manner known per se, and similarly, $Y_1$ alone is cleaved off if $X_1$=Pro.

According to the invention, fusion proteins, which do not contain a formyl methionine group, can be obtained i.a. in one of the following ways:

1) The DNA coding sequence of a suitable prokaryotic N-terminal pro-sequence containing or constituting a signal sequence can be spliced on to the coding sequence of the desired protein at gene level. The DNA sequence corresponding to this pro-sequence may be selected from among the large number of naturally occurring sequences or may be synthetized *in vitro* when their structure at nucleotide and amino acid level is known. After the synthesis of the fusion protein in bacteria, the signal sequence (containing the N-terminal f-Met.) is cleaved off by the bacteria *in vivo*.

2) The DNA coding sequence of the selected pro-sequence is coupled to the coding sequence of the desired protein. The fusion protein (with the N-terminal formyl methionine) synthetized in bacteria, is isolated completely or partly from the cells or the bacterial supernatant. The fusion protein is then treated with aminopeptidase I (AP I) (E.C. 3.4.11.12) from Bacillus stearothermophilus (Roncari & Zuber, Int. J. Protein Research II, 191 (1970)). This amino-peptidase has the same broad specificity as most of the other aminopeptidases, but is moreover able to cleave off N-formyl methionine in the above-mentioned fusion product. This cleavage reaction is to be optimized with respect to time and enzyme concentration as, in case of prolonged incubation, amino-peptidase I can also hydrolyze amino acids of the desired product.

This problem can be reduced by incorporating one or more of the amino acid sequences Gly-Ala, Gly-Gly or Lys-Leu in the pro-sequence because these peptide bonds are hydrolyzed only slowly by the above-mentioned aminopeptidase I.

The DNA coding sequence of the pro-sequence is so formed that the pro-sequence is caused to contain highly active cleavage sites for trypsin (E.C. 3.4.21.4.) or trypsin-like proteases. Such cleavage sites may e.g. consist of the amino acid sequences Arg-Arg, Lys-Lys, Lys-Arg and/or Arg-Lys. After the synthesis of the fusion protein it is isolated *in vivo* wholly or partly, and it treated *in vitro* with trypsin or the trypsin-like protease.

In each of the three above-mentioned procedures it is thus possible according to the invention to provide a protein containing the amino acid sequence corresponding to the desired protein as well as the N-terminal pro-sequence without formyl methionine. In this pro-sequence the C-terminal amino acid, which is bonded directly to the N-terminal·amino acid in the desired protein, must be proline, unless the desired protein itself contains proline as N-terminal or next-to-the-outermost N-terminal amino acid. The pro-sequence may then be removed specifically *in vitro* by treating the fusion protein with a suitable aminopeptidase, e.g. leucine aminopeptidase (E.C. 3.4.11.1). This commercially available aminopeptidase will cleave the sequence, but will stop after having hydrolyzed the bond just before the dipeptide X-Pro. After removal or inactivation of the leucine aminopeptidase, the resulting remaining fusion protein can be treated in one of the following ways if X and optionally Pro are to be cleaved off:

a) X is a different from Gly.

First the amino acid X is cleaved off, e.g. with aminopeptidase P (3.4.11.9) or with prolidase, and then, if desired, proline is cleaved off with proline iminopeptidase (3.4.11.5).

b) X=Gly.

Here Gly-Pro can be cleaved off in the same manner as mentioned under point a). However, when e.g. a Gly-Pro hydrolyzing dipeptidylpeptidase IV (3.4.14.-) is used, whole dipeptides Gly-Pro can be cleaved off in one step.

Example 1

A cloned DNA sequence coding for a protein with an amino acid sequence as in human growth hormone, hGH, (191 amino acid residues of which the first four amino acids are Phe-Pro-Thr-Ile) is coupled with the following synthetically produced, dual-stranded DNA sequence so that the 3' end of the +strand is coupled to the +5' end of the above-mentioned gene, and the 5' end of the −strand of the synthetic DNA sequence is coupled to the 3' end of the above-mentioned gene by blunt end. ligature.

```
+5'   GATCCATGCTGGCTGTAAGC   3'
−3'         TACGACCGACATTCG   5'
```

where the 5 first nucleotides in the +strand are part of a Bam HI restriction site, and the following nucleotide sequences code for the amino acids Met, Leu, Ala, Val, Ser.

The above-mentioned gene is introduced by ordinary gene cloning techniques into an expression plasmide containing a fusioned Trp-Lac promotor as well as the SD sequence AGGA; this structure should therefore express Met-Leu-Ala-Val-Ser-hGH.

This plasmide structure is then introduced into an E. coli cell by prior art techniques. A suitable clone containing the above-mentioned structure is isolated and cultivated in a 30 l scale. The cells are harvested by centrifugation and are suspended in a small volume and lyzated using a so-called "French press".

The expected fusion protein could be demonstrated in the above-mentioned bacterial extract by immunological methods using hGH antibodies.

The fusion product was purified from this extract by hydrophobic interaction chromatography, ammonium sulfate precipitation, gel filtration and anion exchange. Fractions containing the fusion protein were determined immunologically using hGH antibodies.

The purified fusion protein was evaluated to be more than 98% pure by polyacrylic amide gel electrophoresis and ion exchange HPLC. 15 mg were isolated from a 30 l culture. Possible disulfide bridges in the purified fusion protein were reduced and S-carbamidomethylated in principle as described by L. Graf et al.: FEBS Letters *66*(2), 233 (1976). An N-terminal sequence determination according to Edman of the purified and reduced and S-carbamidomethylated fusion protein showed the expected amino acid sequence Met-Leu-Ala-Val-.

This reduced and S-carbamidomethylated product was then treated with leucine aminopeptidase (EC 3.4.11.1) as described by D. H. Sprekman et al., J. Biol. Chem. *212*, 255 (1955) and A. Light: In: Methods in Enzymology, vol. XI, Ed.: C. H. W. Hirs (1967), Academic Press, p. 426. However, the enzymatic hydrolysis was carried out in the presence of urea and aprotinin.

Then the reaction mixture was fractionated by ion exchange, and protein from fractions containing hGH immunoreactivity was isolated. Evaluated by polyacrylic amide gel electrophoresis and ion exchange HPLC the protein was more than 98% pure. N-terminal determination according to Edman showed Phe as the main component.

Example 2

Production of human proinsulin

A gene coding for a protein with an amino acid sequence as in human proinsulin (86 amino acid residues of which the first 4 amino acids are Phe-Val-Asn-Gln), is coupled with the following synthetically produced, dual-stranded DNA sequence so that the 3' end of the +strand is coupled to the 5' end of the +strand of the above-mentioned gene, and the 5' end of the −strand of the synthetic DNA sequence is coupled to the 3' end of the above-mentioned gene by blunt end ligature.

| | | Met | Leu | Val | Ala | Gly | Pro | |
|---|---|---|---|---|---|---|---|---|
| +5' | ATTC | ATG | TTG | GTT | GCT | GGT | CCA | 3' |
| − | G | TAC | AAC | CAA | CGA | CCA | GGT | |

The 5 first nucleotides in the +strand are part of an Eco RI restriction site, and the subsequent nucleotide sequences code for the amino acids Met-Leu-Val-Ala-Gly-Pro.

The above-mentioned gene is introduced by ordinary gene cloning techniques into an expression plasmide containing the promotor for 3-phosphoglycerate kinase and the terminator for the FLP gene, both from S. cerevisiae. (Ref.: Hitzeman et al. (1983), Science 219 620—625).

This plasmide structure is then introduced into an S. cerevisiae cell by prior art techniques. An S. cerevisiae clone containing the above-mentioned structure is isolated and cultivated in a suitable volume. The yeast cells are harvested and lyzated using a so-called "French press".

A protein assumed to be the expected fusion protein is demonstrated in the extract by immunological methods using proinsulin antibodies.

The fusion protein is purified by hydrophobic interaction chromatography.

The fusion protein is purified to >90% purity, evaluated by polyacrylic amide electrophoresis and ion-exchange HPLC. An N-terminal sequence determination according to Edman is carried out to confirm the expected amino acid sequence (corresponding to Met-Leu-Val-Ala-Gly-Pro-Phe-).

This purified product is treated with leucine amino-peptidase (EC 3.4.11.1.) as described by D. H. Sprekman et al., J. Biol. Chem. 212, 225 (1955) and A. Light: In: Methods in Enzymology, vol. VI, Ed.: C. H. W. Hirs (1967), Academic Press, p. 426. Then the reaction mixture is fractionated by ion exchange, and protein from fractions containing proinsulin immunoreactivity is isolated. The purification is better than 90% evaluated by polyacrylic amide electrophoresis and ion-exchange HPLC. Gly-Pro-Phe is found in the isolated product by three consecutive N-terminal degradations according to Edman.

Gly-Pro is removed enzymatically from this product in a manner known per se in a 1-step reaction using post-proline dipeptidyl aminopeptidase (EC 3.4.14.-), cf. R. Walter, W. H. Simmons & T. Yoshimoto: Molecular & Cellular Biochemistry 30(2) (1980), 111—127. Or, alternatively, in a 2-step process, cf. R. I. Delange & E. L. Smith, in: The Enzymes. Ed.: P. D. Boyer, Academic Press (1971), p. 115, to produce human proinsulin.

**Claims**

1. A process for preparing ripe proteins by enzymatic cleavage of a fusion protein with the amino acid sequence:

$$(Y_m \ldots Y_2{-}Y_1){-}(Pro)_p{-}(X_1{-}X_2 \ldots X_n)$$

in which $(Y_m \ldots Y_2{-}Y_1){-}(Pro)_p$ is the pro-sequence, with the proviso that none of the amino acids $(Y_m \ldots Y_2{-}Y_1)$ can be Pro, and the rest is the ripe protein, m is an integer greater than 2, and Y is an arbitrary amino acid, P is 0 if $X_1$ or $X_2$ is Pro, and 1 if $X_1$ or $X_2$ is different from Pro, X is an arbitrary amino acid, and n is an integer equal to or greater than 4, characterized by carrying out the enzymatic cleavage by stepwise cleavage off of the amino acid groups $Y_m \ldots Y_2$ if p=1 or $X_1$=Pro, or the groups $Y_m \ldots Y_2{-}Y_1$ if $X_2$=Pro, by means of an aminopeptidase, and then the two amino acids $Y_1$-Pro if p=1 are cleaved off enzymatically in one or two steps in a manner known per se, and similarly $Y_1$ alone is cleaved off if $X_1$=Pro.

2. A process according to claim 1, characterized in that the aminopeptidase is leucine aminopeptidase.

3. A process according to claim 1 or 2, characterized in that the fusion protein includes the amino acid sequence occurring in a human growth hormone.

4. A process according to claim 1 or 2, characterized in that the fusion protein contains proinsulin.

**Patentansprüche**

1. Verfahren zur Herstellung reifer Protein durch enzymatische Spaltung eines Fusionsproteins mit der Aminosäuresequenz

$$(Y_m \ldots Y_2{-}Y_1){-}(Pro)_p{-}(X_1{-}X_2 \ldots X_n)$$

worin

$(Y_m. . .Y_2—Y_1)—(Pro)_p$ die Pro-Sequenz ist, mit der Maßgabe, daß keine der Aminosäuren $(Y_m. . .Y_2—Y_1)$ Pro sein kann, und der Rest das reife Protein ist, m eine ganze Zahl größer als 2 ist und Y eine beliebige Aminosäure ist, p 0 ist, falls $X_1$ oder $X_2$ Pro ist, und 1 ist, falls $X_1$ oder $X_2$ verschieden von Pro ist, X eine beliebige Amminosäure ist und n eine ganze Zahl gleich oder größer als 4 ist, dadurch gekennzeichnet, daß enzymatische Spaltung durch schrittweise Abspaltung der Aminosäure-Gruppen $Y_m. . .Y_2$, falls p=1 oder $X_1$=Pro, oder der Gruppen $Y_m. . .Y_2—Y_1$, falls $X_2$=Pro, mittels einer Amino-peptidase erfolgt und danach die beiden Aminosäuren $Y_1$-Pro, falls p=1, enzymatisch in einem Schritt oder in zwei Schritten in an sich bekannter Weise abgespalten werden und in ähnlicher Weise $Y_1$ allein abgespalten wird, falls $X_1$=Pro.

2. Verfahren nach Anspruch 1, dadurch gekennzeichnet, daß die Aminopeptidase Leucin-Aminopeptidase ist.

3. Verfahren nach Anspruch 1 oder 2, dadurch gekennzeichnet, daß das Fusionsprotein die in einem Human-Wachstumshormon vorkommende Aminosäure-Sequenz enthält.

4. Verfahren nach Anspruch 1 oder 2, dadurch gekennzeichnet, daß das Fusionsprotein Proinsulin enthält.

**Revendications**

1. Procédé de préparation de protéines matures par clivage enzymatique d'une protéine de fusion ayant la séquence d'amino-acides:

$$(Y_m. . . Y_2—Y_1)—(Pro)_p—(X_1—X_2. . .X_n)$$

dans laquelle $(Y_m. . . Y_2—Y_1)—(Pro)_p$ est la pro-séquence, sous réserve qu'aucun des amino-acides $(Y_m. . .Y_2—Y_1)$ ne puisse être Pro, et le reste est la protéine mature, *m* est un nombre entier supérieur à 2, et Y est un amino-acide arbitraire, *p* est égal à 0 si $X_1$ ou $X_2$ est Pro et est égal à 1 si $X_1$ ou $X_2$ est différent de Pro, X est un amino-acide arbitraire, et *n* est un nombre entier égal ou supérieur à 4, caractérisé en ce qu'il consiste à effectuer le clivage enzymatique en éliminant par clivage progressif les groupes d'amino-acides $Y_m. . . Y_2$ si *p*=1 ou $X_1$=Pro, ou les groupes $Y_m. . .Y_2—Y_1$ si $X_2$=Pro, au moyen d'une aminopeptidase, puis si *p*=1, en éliminant par clivage enzymatique en une ou deux étapes d'une manière bien connue les deux amino-acides $Y_1$-Pro, et en éliminant de manière similaire par clivage $Y_1$ seul si $X_1$=Pro.

2. Procédé suivant la revendication 1, caractérisé en ce que l'aminopeptidase est la leucine-amino-peptidase.

3. Procédé suivant la revendication 1 ou 2, caractérisé en ce que la protéine de fusion comprend la séquence d'amino-acides présente dans une hormone de croissance humaine.

4. Procédé suivant la revendication 1 ou 2, caractérisé en ce que la protéine de fusion contient de la pro-insuline.